# EUROPEAN PATENT SPECIFICATION

(11) **EP 3 000 473 B1**
(45) Date of publication and mention of the grant of the patent: **05.07.2017**
(21) Application number: 15183798.6
(22) Date of filing: 04.09.2015
(51) Int. Cl.: A61K 35/745, A61K 35/747, A61K 35/744, A61P 31/04, A61P 31/10

(54) **PROBIOTIC COMPOSITION FOR USE IN THE PREVENTION OF BIRTH-ACQUIRED BACTERIAL AND FUNGAL INFECTIONS IN NEWBORNS**
PROBIOTISCHE ZUSAMMENSETZUNG ZUR VERWENDUNG BEI DER VORBEUGUNG VON BEI DER GEBURT ERWORBENEN BAKTERIELLEN UND PILZINFEKTIONEN BEI NEUGEBORENEN
COMPOSITION PROBIOTIQUE DESTINÉE À ÊTRE UTILISÉE DANS LA PRÉVENTION D'INFECTIONS BACTÉRIENNES ET FONGIQUES CHEZ LES NOUVEAU-NÉS

(30) Priority: 23.09.2014 IT MI20141650
(43) Date of publication of application: 30.03.2016
(73) Proprietor: Velleja Research SRL, 29010 Pontenure (PC) (IT)
(72) Inventor: DI PIERRO, Francesco, 29010 PONTENURE (PC) (IT)
(74) Representative: Bianchetti Bracco Minoja S.r.l.

(56) References cited:
- EP-A1- 0 904 784
- Francesco Di Pierro: "Probiotici specifici in gravidanza per pilotare la colonizzazione intra-partumdel nascituro", L'INTEGRATORE NUTRIZIONALE, vol. 17, no. 4 15 January 2015 (2015-01-15), pages 2-6, XP002736068, Retrieved from the Internet: URL:http://www.vellejaresearch.com/upload/ documenti/226_20141208191553.pdf [retrieved on 2015-02-16]
- Anonymous: "iNatal® sachets", Omeopiacenza , 16 February 2015 (2015-02-16), XP002736069, Retrieved from the Internet: URL:http://www.omeopiacenza.it/en/product- details.php?p=inatal-bustine [retrieved on 2015-02-17]
- LI JINGJIE ET AL: "Lactococcus lactis expressing food-grade [beta]-galactosidase alleviates lactose intolerance symptoms in post-weaning Balb/c mice.", APPLIED MICROBIOLOGY AND BIOTECHNOLOGY, vol. 96, no. 6, December 2012 (2012-12), pages 1499-1506, XP035139565, ISSN: 1432-0614
- ERMOLENKO E I ET AL: "[Influence of probiotic enterococci on the growth of Streptococcus agalactiae].", ZHURNAL MIKROBIOLOGII, EPIDEMIOLOGII, I IMMUNOBIOLOGII, no. 5, September 2007 (2007-09), pages 73-77, XP009182644, ISSN: 0372-9311
- CHATTHA KULDEEP S ET AL: "Divergent immunomodulating effects of probiotics on T cell responses to oral attenuated human rotavirus vaccine and virulent human rotavirus infection in a neonatal gnotobiotic piglet disease model.", JOURNAL OF IMMUNOLOGY (BALTIMORE, MD. : 1950), vol. 191, no. 5, 1 September 2013 (2013-09-01), pages 2446-2456, XP002736072, ISSN: 1550-6606
- LO SKIAVO L A ET AL: "[Dynamics of contamination and persistence of Clostridium difficile in intestinal microbiota in newborn infants during antibiotic therapy and use of probiotic strain enterococcus faecium L3].", ANTIBIOTIKI I KHIMIOTERAPIIA = ANTIBIOTICS AND CHEMOTERAPY [SIC] / MINISTERSTVO MEDITSINSKOI I MIKROBIOLOGICHESKOI PROMYSHLENNOSTI SSSR, vol. 58, no. 11-12, 2013, pages 13-18, XP009182646, ISSN: 0235-2990
- DATABASE GNPD [Online] MINTEL; June 2013 (2013-06), "Biotiflor Advanced Capsules", XP002736074, Database accession no. 2098722
- DOTTERUD C K ET AL: "Probiotics in pregnant women to prevent allergic disease: a randomized, double-blind trial.", THE BRITISH JOURNAL OF DERMATOLOGY, vol. 163, no. 3, September 2010 (2010-09), pages 616-623, XP055170170, ISSN: 1365-2133
- AZAD MEGHAN B ET AL: "Probiotic supplementation during pregnancy or infancy for the prevention of asthma and wheeze: systematic review and meta-analysis.", BMJ (CLINICAL RESEARCH ED.), vol. 347, F6471, 2013, pages 1-15, XP002736076, ISSN: 1756-1833, DOI: 10.1136/bmj.f6471
- DE VRESE MICHAEL ET AL: "Probiotics and Prebiotics: Effects on Diarrhea", THE JOURNAL OF NUTRITION, vol. 137, no. 3, March 2007 (2007-03), pages 803S-811S, XP002736077,
- COW'S MILK ALLERGY MODIFIED BY ELIMINATION AND LACTOBACILLI STUDY GROUP ET AL: "The acquisition of tolerance toward cow's milk through probiotic supplementation: A randomized, controlled trial", JOURNAL OF ALLERGY AND CLINICAL IMMUNOLOGY, vol. 121, no. 6, 1 June 2008 (2008-06-01), pages 1448-1454, XP022760256, ELSEVIER, AMSTERDAM, NL ISSN: 0091-6749, DOI: 10.1016/J.JACI.2008.03.018
- SCHULTZ MICHAEL ET AL: "Administration of oral probiotic bacteria to pregnant women causes temporary infantile colonization", JOURNAL OF PEDIATRIC GASTROENTEROLOGY AND NUTRITION, vol. 38, no. 3, 1 March 2004 (2004-03-01), pages 293-297, XP009102393, LIPPINCOTT WILLIAMS WILKINS, INC, US ISSN: 0277-2116, DOI: 10.1097/00005176-200403000-00012
- Mikkel Jungersen: "Study summaries BB-12®", Chr. Hansen A/S , October 2013 (2013-10), XP002736078, Retrieved from the Internet: URL:http://cdn.chr-hansen.com/uploads/tx_t cdownloadablessecure/Selected_summaries_Bi fidobacterium_BB-12_chr_hansen.pdf [retrieved on 2015-02-17]
- Sandy Smith: "Lactobacillus Rosell-215", Institut Rosell-Lallemand , 12 July 2013 (2013-07-12), XP002736079, Retrieved from the Internet: URL:https://web.archive.org/web/2013071209 4954/http://www.probacti.de/files/6_DOWNLO AD%20Lactobacillus%20Rosell%20215.pdf [retrieved on 2015-02-17]
- VRESE M DE ET AL: "Probiotics, prebiotics, and synbiotics", ADVANCES IN BIOCHEMICAL ENGINEERING, BIOTECHNOLOGY, vol. 111, 1 January 2008 (2008-01-01), pages 1-66, XP008108606, SPRINGER, BERLIN, DE ISSN: 0724-6145, DOI: 10.1007/10_2008_097

## Description

### SUMMARY OF THE INVENTION

The present invention relates to a probiotic composition comprising *Enterococcus faecium* L3, *Lactococcus lactis* SP38, *Lactobacillus casei* RO215 and *Bifidobacterium animalis lactis* BB12, for use in the prevention of birth-acquired bacterial and fungal infections in newborn infants.

### TECHNICAL BACKGROUND

Mother-to-child interaction is characterised by the possible vertical, one-way passage of infectious agents to the newborn baby. Some pathogens can be transmitted from mother to child as a result of the development of a primary, recurrent or chronic infection by the mother. Subclinical infections or carrier status can also be the source of passage of pathogens from mother to child. This type of mother-to-child transmission can take place *in utero* (congenital infection), during childbirth (perinatal infection), or through breastfeeding (postnatal infection). Said transmission can have numerous consequences, some of which can be very serious: miscarriage, foetal death, delayed intrauterine growth, congenital abnormalities, and neonatal or postnatal disorders of various extents. A number of risk factors seem to influence the incidence of mother-to-child transmission. In addition to the presence of full-blown infections in the mother, the mother's pathogen count, the type of infection (primary or recurrent), duration of rupture of membranes, method of childbirth, socioeconomic status and type of feeding also influence the vertical passage of pathogens. Nowadays, mother-to-child transmission of many viruses can be prevented by the use of vaccines, passive immunisation and antivirals. The risk of transmission of perinatal and postnatal viral infections can also be reduced if the birth takes place by caesarean section or the mother does not breastfeed. However, in the case of vertical mother-to-child transmission of bacteria and fungi, the situation appears to be complicated by the absence of available vaccines and the fact that most antibiotics and antifungals present an evident teratogenic risk, and therefore cannot be administered until after the birth. Examples of vertical infections of this kind are infections caused by *Streptococcus agalactiae.*

*Streptococcus agalactiae* (Group B beta-haemolytic streptococcus, otherwise known as GBS) is the main cause of neonatal bacterial infection in the developed countries. GBS is a Gram-positive bacterium which is primarily located in the gastrointestinal and genitourinary tract. The symptoms of neonatal GBS infection include pneumonia, respiratory difficulty and meningitis. The baby is usually colonised during its passage through the birth canal. The mother, when colonised, is usually asymptomatic. The bacterium is present in the vaginas of about 15-20% of women towards the end of pregnancy. During the first 7 days of life (early-onset infection), about 3% of colonised babies develop infection, especially meningitis, which can be fatal or leave after-effects; this infection mainly derives from vertical transmission of GBS during the birth. A GBS infection that arises between the first week and the first 90 days of the baby's life (late-onset infection) more probably derives from transmission at a post-neonatal stage. In Italy, as required by national guidelines, all female patients undergo vaginal and rectal screening to test for GBS infection. This test is conducted between the 36th and 37th week of pregnancy. If the culture test is positive, both mother and child are treated with antibiotic prophylaxis during labour. The baby's discharge from hospital is often postponed until it is ten days old. Similar approaches are taken today in most economically developed countries. Antibiotic prophylaxis during labour and until birth reduces the risk of early-onset neonatal infection to around 0.4-0.5%. Penicillin G is the preferred antibiotic. If penicillin is contraindicated, the preferred antibiotics are erythromycin or clindamycin, which are not considered to present any particular risk to the baby.

Another possible vaginal infection, which is less important in terms of consequences for the baby's health, but problematic because it often requires the mother to be treated with medicaments that are potentially dangerous to the baby, is caused by various species of Candida (*albicans, kruzei, glabrata,* etc.).

### Subject of the invention

The invention is described in the claims. It has been observed that oral administration during pregnancy of a particular probiotic composition, consisting of *Enterococcus faecium* L3, *Lactococcus lactis* SP38, *Lactobacillus casei* R215 and *Bifidobacterium animalis lactis* BB12, reduces positivity to the (vaginal and rectal) culture test for *Streptococcus agalactiae* by 50% and the possibility of vaginal Candida infection by 66%.

The present invention therefore relates to a probiotic composition comprising *Enterococcus faecium* L3, *Lactococcus lactis* SP38, *Lactobacillus casei* RO215 and *Bifidobacterium animalis lactis* BB12, for use in the prevention of birth-acquired bacterial and fungal infections in newborn infants. The strain *Enterococcus faecium* L3 is available from the BCCM/LMG Bacteria Collection (Gent, Belgium) under number P-27496.

The strain *Lactococcus lactis ssp.* SP 38 is available from DSM (Deutsche Sammlung von Mikroorganismen) under number 26868.

The strain *Lactobacillus casei* RO215 is available from CNCM (Collection Nationale de Cultures de Microorganismes, Institut Pasteur, France) under number 1-3429.

The strain *Bifidobacterium animalis subsp. lactis* BB12 is available from DSM under number 15954.

The strains used in the composition for use according to the invention have proved effective when administered at a dose ranging between 10,000 CFU/dose/day and 100,000,000,000/CFU/dose/day. The composition for use according to the invention will therefore comprise strains of *Enterococcus faecium* L3, *Lactococcus lactis* SP38, *Lactobacillus casei* RO215 and *Bifidobacterium animalis lactis* BB12 in quantities such as to provide a daily dose preferably ranging between 10,000 CFU/dose and 100,000,000,000/CFU/dose.

The composition for use according to the invention was administered to 50 pregnant women from the 28th to the 36th week of pregnancy, at the end of which period a vaginal and rectal swab was taken to establish whether the mother tested positive for Group B streptococcus. Only 5 women tested positive. Conversely, in the control group treated with the placebo, 10 women tested positive for Group B streptococcus. The product was still administered until the day of the birth.

The composition for use according to the invention was also administered to 50 pregnant women from the 28th to the 40th week of pregnancy (day of birth); vulvovaginitis caused by *Candida albicans* (or other species) was found in 15 women. Conversely, in the group treated with the placebo, the infections were found in 45 women.

According to a further aspect described herein, the compositions in question can also include other ingredients with a complementary or useful action for the purposes of the invention, selected, for example, from vitamins (Group B, C, D, E and K), mineral salts (Mg, K, Ca, P, I, Mn and F), herbal extracts containing flavonoids/polyphenols (from vine, citrus fruit, tea, ginkgo, milk thistle, cranberry or bilberry), herbal extracts containing terpenes or saponins (centella or ginseng) or similar, or amino acids.

The compositions for use according to the invention could be formulated suitably for oral administration, and prepared according to conventional methods well known in pharmaceutical technology, such as those described in Remington's Pharmaceutical Handbook, Mack Publishing Co., N.Y., USA, using conventional excipients acceptable for their final use.

Examples of formulations for use to the invention are set out below.

### Formulation examples

### EXAMPLE 1: 3 g STICK

| | | |
|---|---|---|
| *Enterococcus faecium* L3 | 20 bln cfu/g | 500 mg |
| *Lactococcus lactis* SP 38 | 100 bln cfu/g | 100 mg |
| *Lactobacillus casei* RO215 | 100 bln cfu/g | 100 mg |
| *Bifidobacterium animalis subsp. lactis* | 30 bln cfu/g | 133.3 mg |
| Maltodextrin | | 1166.7 mg |
| Silica | | 500 mg |
| Milk flavouring | | 500 mg |

### EXAMPLE 2: 2 g TABLETS

| | | |
|---|---|---|
| *Enterococcus faecium* L3 | 20 bln cfu/g | 500 mg |
| *Lactococcus lactis* SP 38 | 100 bln cfu/g | 100 mg |
| *Lactobacillus casei* RO215 | 100 bln cfu/g | 100 mg |
| *Bifidobacterium animalis subsp. lactis* | 30 bln cfu/g | 133.3 mg |
| Maltodextrin | | 666.7 mg |
| Vanilla flavouring | | 500 mg |

### EXAMPLE 3: 1 g TABLETS

| | | |
|---|---|---|
| *Enterococcus faecium* L3 | 10 bln cfu/g | 250 mg |
| *Lactococcus lactis* SP 38 | 50 bln cfu/g | 50 mg |
| *Lactobacillus casei* RO215 | 50 bln cfu/g | 50 mg |
| *Bifidobacterium animalis subsp. lactis* | 10 bln cfu/g | 50 mg |
| Maltodextrin | | 350 mg |
| Strawberry flavouring | | 250 mg |

## Claims

1. Probiotic composition comprising *Enterococcus faecium L3, Lactococcus lactis SP38, Lactobacillus casei RO215* and *Bifidobacterium animalis lactis* BB12, for use in the prevention of birth-acquired bacterial and fungine infections in the newborn infant.

2. Composition for the use according to claim 1, wherein the infections are caused by *Streptococcus agalactiae.*

3. Composition for the use according to claim 1, wherein the infections are caused by Candida sp.

4. Composition for use according to claim 1 further comprising vitamins and/or salts and/or vegetal extracts and/or amino acids.

## Patentansprüche

1. Probiotische Zusammensetzung, umfassend *Enterococcus faecium L3, Lactococcus lactis SP38, Lactobacillus casei RO215* und *Bifidobacterium animalis lactis BB12,* zur Verwendung bei der Prävention von bei der Geburt erworbenen bakteriellen Infektionen und Pilzinfektionen bei dem neugeborenen Säugling.

2. Zusammensetzung zur Verwendung gemäß Anspruch 1, wobei die Infektionen durch *Streptococcus agalactiae* verursacht werden.

3. Zusammensetzung zur Verwendung gemäß Anspruch 1, wobei die Infektionen durch Candida sp. verursacht werden.

4. Zusammensetzung zur Verwendung gemäß Anspruch 1, ferner umfassend Vitamine und/oder Salze und/oder pflanzliche Extrakte und/oder Aminosäuren.

## Revendications

1. Composition probiotique comprenant *Enterococcus faecium L3, Lactococcus lactis SP38, Lactobacillus casei RO215 et Bifidobacterium animalis lactis* BB12, destinée à une utilisation dans la prévention des infections bactériennes et fongiques acquises à la naissance par le nouveau-né.

2. Composition destinée à une utilisation selon la revendication 1, les infections étant causées par *Streptococcus agalactiae.*

3. Composition destinée à une utilisation selon la revendication 1, les infections étant causées par *Candida* sp.

4. Composition destinée à une utilisation selon la revendication 1 comprenant en outre des vitamines et/ou des sels et/ou des extraits végétaux et/ou des acides aminés.
